# EUROPEAN PATENT APPLICATION

(11) **EP 0 800 830 A2**
(43) Date of publication of application: **15.10.1997**
(21) Application number: 97107677.3
(22) Date of filing: 18.10.1990
(51) Int. Cl.: A61K 48/00, A61K 9/127, A61K 47/48

(54) **Method of in vivo delivery of functioning foreign genes**

(30) Priority: 03.11.1989 US 431552
(62) Divisional of application: 90916560.7
(71) Applicant: VANDERBILT UNIVERSITY, Nashville, TN 37240 (US)
(72) Inventor: Brigham, Kenneth L., Nashville, TN 37201 (US)
(74) Representative: VOSSIUS & PARTNER

(57) **Abstract**

A method of expressing a foreign gene in cells of a mammalian organ in vivo includes the step of injecting genetic material capable of inducing desired gene products into a mammal. The genetic material is complexed to cationic liposome carriers. The genetic material is lipofected into cells of the mammalian organ. Gene expression and cellular function of the genetic material are activated to produce the desired foreign products in the cells of the organ.

## Description

### TECHNICAL FIELD

The present invention relates to the incorporation of a foreign gene into cells of a mammalian organ in vivo. More specifically, the present invention provides in vivo transient transfection of lung cells by intravenous injection of a genetic material.

### BACKGROUND ART

Functioning DNA can be introduced by a variety of techniques resulting in either transient expression of the genes of interest, referred to as transient transfection, or permanent transformation of the host cells resulting from incorporation of the foreign DNA into the host genome. Berger, S.L., and A.R. Kimmel. 1987. "Guide to molecular cloning techniques". Methods in Enzymology. 152:692-694. Potter, H., L. Weir and P. Leder. 1984. "Enhancer-dependent expression of human K immunoglobulin genes introduced into mouse pre-B lymphocytes by electroporation". Proc. Natl. Acad. Sci. 81:7161-7165. Plasmids complexed with cationic liposomes, referred to as liposome-DNA complexes, have been used for the transfection of cell cultures.

The use of liposomes for such transfection has been referred to as lipofection. Of the many techniques used for transfecting cells, lipofection has been found to offer advantages of simplicity and high transfection efficiency. Brigham, K.K., B. Meyrick, B. Christman, L.C. Berry, and G. King. "Expression of a prokaryotic gene in cultured lung endothelial cells following lipofection with a plasmid vector". Am. J. Resp. Cell. Mol. Biol., 1:95-100, 1989. Felgner, P.L., T.R. Gadek, M. Holm, R. Roman, H.W. Chan, M. Wenz, J.P. Northrop, G.M. Rignold, and M. Danielsen, 1987. "Lipofection: A highly efficient, lipid-mediated DNA-transfection procedure". Proc. Natl. Acad. Sci. 84:7413-7417.

Kaneda et al, Sci. Vol. 243, January 1989, pages 375-378 discloses an in vivo transfection technique wherein plasmid-DNA and nuclear protein were cointroducted into nondividing cells in rat liver by injection into the portal veins of adult rats, the plasmid-DNA being carried into liver cell nuclei by the nuclear protein. Wu et al in the Journal of Biological Chemistry, Vol. 263 No. 29 issue of Oct. 15, pages 14621-14624, 1988 disclose an in vivo transfection technique wherein plasmids are bound to ligands for specific hepatic galactose receptor complexes injected IV and showed specific gene expression in the liver.

The present invention provides an in vivo lipofection technique resulting in expression of desired foreign products in cells of a mammalian organ.

### SUMMARY OF THE INVENTION

In accordance with the present invention, there is provided a method of expressing a foreign gene in cells of a mammalian organ in vivo, the method including the steps of injecting a gene expression inducer of desired gene products into a mammal. The inducer is complexed to a cationic liposome carrier. The inducer is lipofected into cells of the mammalian organ. Gene expression and cellular function of the inducer is activated to produce the desired gene products in the cells of the organ.

### FIGURES IN THE DRAWINGS

Other advantages of the present invention will be readily appreciated as the same becomes better understood by reference to the following detailed description when considered in connection with the accompanying drawings wherein:
Figure 1 shows expression of a human growth hormone gene in the lungs of mice following intravenous injection of a plasmid containing the coding region for human growth hormone driven by a metallothionein promoter. Data are expressed as ng hGH per 24 hours per gm tissue and all points are means of data from 2 different animals. Control values were subtracted from the data in the figure. There was minimal expression of the foreign gene in either kidneys or liver, but the gene was expressed in the lungs with a time course similar to that seen in cultured endothelial cells;
Figure 2 shows CAT activity in the organs of mice 72 hours following injection of the DNA-liposome complex; and
Figure 3 shows lung CAT activity for the 3 days following injection of the liposome-DNA complex.

### DETAILED DESCRIPTION OF THE INVENTION

Generally, the present invention provides a method of expressing a foreign gene in cells of a mammalian organ in vivo. The method includes the steps of injecting a gene expression inducer of desired gene products into a mammal. For example, the gene expression inducer could be genetic material, such as DNA or RNA. The gene products could be, for example, a polypeptide or protein such as a growth hormone.

The inducer is complexed to a cationic liposome carrier. An example of such a carrier is LIPOFECTIN-, manufactured by Bethesda Research Laboratories Life Technologies, Inc. of Gaithersburg, MD. LIPOFECTIN- reagent has previously been used for transfecting cells in vitro. LIPOFECTIN- reagent contains the cationic lipid LN-[1-(2,3-Dioleyloxy) propyl]-N,N,N-trimethylammonium chloride which complexes with the DNA by ionic interaction. Generally, the complex then fuses with cell membrane and is transfected into the cell. Methods of utilizing lipofection as highly efficient transfection procedure have been reported by Felgner et al, Proc. Natl. Acad. Sci. U.S.A. Vol. 84, pages 7413-7417 Nov. 1987; and by Felgner et al Nature Vol. 337 26 Jan. 1989, pages 387-388.

Utilizing the cationic liposome carrier, the genetic material is lipofected into the cells of the mammalian organ. Gene expression and cellular function of the genetic material is activated to produce the desired foreign products in the cells of the organ. That is, expression of particular expression vectors of the genetic material can be promoted.

More specifically, plasmid bound to the carrier, the plasmid containing the predetermined genetic material, is intravenously injected into the blood stream of the mammal in proximity to the organ for transfection. It has been found that when such plasmid carrier complexes are injected intratracheally, a high degree of transfection occurs in the cells of the lung. The genetic expression is transient. The transient expression in vivo of the transfected gene provides utility for gene therapy, including prevention and treatment of acute or subacute conditions which are not necessarily related to any genetic abnormality. The acute abnormality could be irradicated by the transient treatment, prolonged treatment not being necessary. Hence, the present invention could be used not only as a tool for correcting genetic abnormalities, but also as new category of therapy which could broadly be applicable to human desease states. Accordingly, there is an advantage to the transiency of the plasmid transfection.

### EXPERIMENTAL DATA

### 1. Expression of Coding Region for Human Growth Hormone.

### Methods

### Plasmid Descriptions

Plasmid constructs obtained from Nichols Institute Diagnostics, San Juan Capistrano, California, were used. The plasmid, PXGH5, was a 6.7 kilobase construct in pUC12 containing the coding region for human growth hormone driven by a mouse metallothionein (mMT-1) promoter. Plasmid DNA was propagated in Escherichia coil. Plasmid DNA was isolated by alkaline lysis and purified by isopyknic equilibration gradient centrifugation in cesium chloride and ethidium bromide. Maniatis, T., E.F. Fritsch, and J. Sam brook. 1982. Molecular Cloning. A Laboratory Manual. Cold Spring Harbor: Cold Spring Harbor Laboratory.

### In vivo Transfection

In vivo studies were carried out in 6 week old specific pathogen free International Cancer Research/Harlan Sprague-Dawley female mice (body weight 20-25 gm). Beginning 24 hours prior to DNA injection and continuing to the end of the experiment, all mice were given 5000 ppm ZnSO₄ in their drinking water. This amount of zinc has been shown to activate the metallothionein promoter in transgenic mice. Palmiter, R.D., R.L. Brinster, R.E. Hammer, M.E. Trumbauer, M.G. Rosenfeld, N.C. Birnberg, and R.M. Evans. 1982. Dramatic growth of mice that develop from eggs microinjected with metallothionein-growth hormone fusion genes. Nature 300:611-615. Each mouse was injected intravenously through a tail vein puncture with a 25 gauge needle with 30 ug pXGH5 DNA complexed to 150 ug liposome (Lipofectin TM) in a total volume of 300 ul. Mice were killed 1, 3 and 5 days following DNA injection by an intraperitoneal injection of pentobarbital and the lungs, liver and kidneys were removed under sterile conditions. The organs were weighed, minced and placed in 60 mm Petri dishes to which were added 2ml medium 199. The dishes were incubated for 24 hours at 37'C in 5% CO₂ after which the contents were centrifuged and growth hormone assays were performed on the medium. Growth hormone production was calculated as the product of hGH concentration and the total volume of medium (2ml), normalized to organ weight and expressed as ng hGH per gm tissue per 24 h. Identical measurements were made in 5 non-transfected control mice.

### 2. Results

### In vivo Transfection

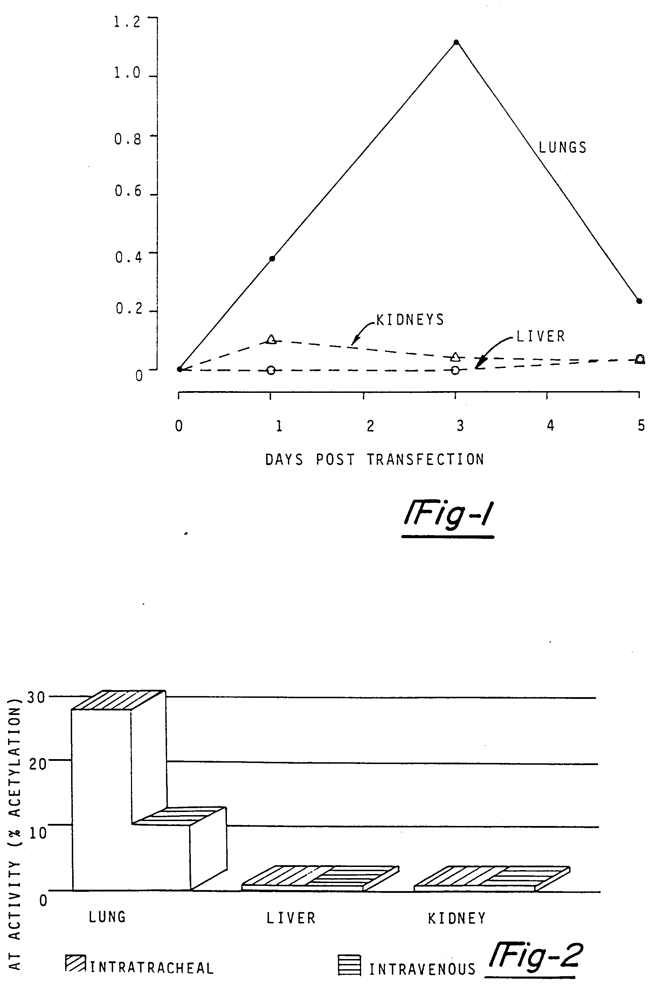

Medium from 24 hour incubations of lungs, liver and kidneys removed from mice which were not injected with the plasmid-liposome complex showed very low levels of immunoreactive hGH. Calculated hGH production in ng per gm tissue per 24 hours was: lungs = 0.26; kidneys = 0.26; liver = 0.06 (mean of N = 5 in all cases). Expression of the hGh gene in mouse organs removed at different intervals following intravenous injection of plasmid-liposome complex is shown in Figure 1. There was little production of growth hormone by either kidneys or liver. However, lungs removed from the transfected mice produced substantial amounts of growth hormone. hGh production was increased by 24 hours after injection, peaked at 3 days after injection and by 5 days was declining. This time course is similar to that seen in cultured endothelial cells.

### 3. Experiment 2

A plasmid which contained a prokaryotic (bacterial) gene, chloramphenicol acetyltransferase (CAT), was bound to LIPOFECTIN- reagent. The liposome-DNA complex was injected into mice. The lungs of the mice were shown to express CAT gene. CAT is not present in any mammalian cells normally and measurement of CAT activity is thus an absolute index of successful expression of the gene. Previously, this plasmid construct was commonly used in cultured cells where CAT activity is proportional to mRNA levels.

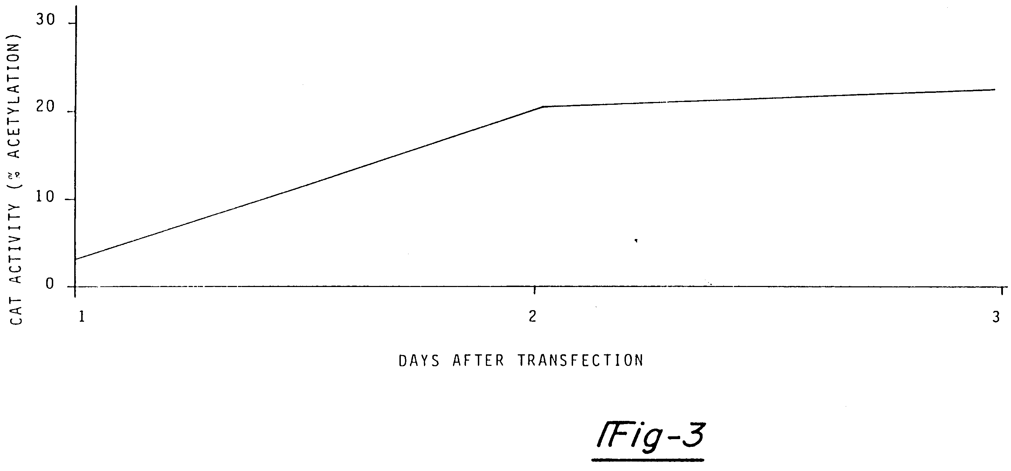

Figure 2 shows CAT activity in the organs of mice 72 hours following injection of the DNA-liposome complex and Figure 3 shows lung CAT activity for the three days following intravenous injection of the lipsome-DNA complex. There is a large amount of activity in the lungs with no detectable activity in peripheral organs. It is expected that the specific transfection of the lungs is probably because of the lungs being the first organ distal to the site of injection. When the DNA-liposome complex is given intratracheally, as indicated in the Figure, larger amounts of activity appear in the lungs than with intravenous studies. Intraperitoneal injection show no CAT activity in any organ up to 6 days following injection.

### 4. Discussion

The present inventive method permits introduction of foreign genes directly into host cells by injection of the genetic material. DNA bound to specifically synthesized cationic liposomes can introduce plasmids and other gene vectors into cultured cells with high efficiency. It is suspected that the plasmid does not enter the host genome and does not replicate in a mammalian cell so that the gene expression is transient, in the present examples lasting for greater than a week.

Following intravenous injection of the DNA-liposome complex, the major expression of the gene was found in the lungs. This is interpreted to mean that the principal organ transfected following intravenous injection is the first capillary bed down stream from the injection site. Accordingly, it should be possible to selectively transfect organs by injecting the liposome DNA complex in the artery supplying the organ.

Previously, Iannuzzi and Associates transfected human airway eipthelial cells with a plasmid containing the prokaryotic gene, CAT, driven by an SV40 promoter by electroporation. Iannuzzi, M.C. , J.L. Weber, J. Yankaskas, R. Boucher, and F.S. Collins. 1988. "The introduction of biologically active foreign genes into human respiratory epithelial cells using electroporation". AM. Rev. Resp. Dis. 138:965-968. These experiments showed expression of the CAT gene in transfected cells. Zwiebel and co-workers transformed rabbit aorta endothelial cells in culture by infecting them with a retroviral vector containing either hGH or adenosine deaminase coding regions driven by an SV40 promoter. Zwiebel, J.A., S.M. Freeman, P.W. Kantoff, K. Cornetta, U.S. Ryan, and W.F. Anderson. 1989. "High-level recombinant gene expression in rabbit endothelial cells transduced by retroviral vectors". Science 243:220-243. The experiment showed expression of the genes in the transformed cells which persisted through many generations in culture. The inventors of the present invention earlier showed that bovine lung endothelial cells could be transfected by lipofection at very high efficiency with a plasmid containing the CAT coding region driven by a Rous sarcoma virus promoter. Brigham, K.L., B. Meyrick, B. Christman, L.C. Berry, and G. King. "Expression of a prokaryotic gene in cultured lung endothelial cells following lipofection with a plasmid vector". Am. J. Resp. Cell Mol. Biol. 1:95-100, 1989.

The studies detailed above demonstrate the use of transfecting the lung of intact animals with a functioning gene encoding a physiologically relavent secreted human protein driven by the metallothionein promoter. The time course of expression of the transfected gene in the lung was similar to the time course of expression over the same gene in cultured lung endothelial cells (experiments not reported).

Gene therapy has generally been conceived as principally applicable to genetic deficiency diseases where permanent cure may be effected by introducing a functioning gene. Freiedmann, T. 1989. "Progress toward human gene therapy". Science 244:1275-1281. However, a much larger group of deseases might be treatable by transiently engineering host cells to produce beneficial proteins. For example, the intracellular enzymes, superoxide dismutase and catalase, may be crucial protectors of the lungs from oxidant injury and increased intracellular levels of these proteins might protect the lung from injury of a variety of causes. Transient increase in endothelial prostaglandin synthetase resulting in increased production of prostacyclin and prostaglandin E₂ might be beneficial in several deseases. The secreted antiprotease, alpha-1 antirypsin, may be important in the pathogenesis of emphysema and other lung deseases and genetic engineering of lung cells to produce this protein could be therapeutic in human desease states. Garver, R.I., J.F. Mornex, T. Nukiwa, M. Brantly, M. Courtney, J.P. CeCoco, and R.G. Crystal. 1986. "Alpha-1 antritrypsin genes". N. Engl. J. Med. 314:762-766. Numerous other theoretical possibilities could be suggested.

The present data demonstrate that lung cells in living animals can be engineered by transfection to express foreign genes encoding both intracellular and secreted proteins. Several promoters, including those which permit experimental control of gene expression, can be used to drive expression of the transfected gene. Use of plasmid vectors helps to assure transient expression of the gene. This approach will permit elucidation of molecular mechanisms effecting gene expression in specific lung cells and specific investigation of the rolls of various proteins in lung cell responses. In addition, in vivo transient transfection may make gene therapy applicable to a broad range of human deseases.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the appended claims wherein reference numerals are merely for convenience and are not to be in any way limiting, the invention may be practiced otherwise than as specifically described.

## Claims

1. A method of expressing a foreign gene in cells of a mammalian organ in vivo, said method including the steps of: injecting a gene expression inducer of desired gene products into a mammal, the inducer being complexed to a cationic liposome carrier; lipofecting the inducer into cells of the mammalian organ; and activating gene expression and cellular function of the inducer to produce the desired foreign gene products in cells of the organ.

2. A method as set forth in claim 1 wherein said injecting step is further defined as injecting a plasmid containing the inducer and bound to the carrier into the mammal.

3. A method as set forth in claim 2 wherein said injecting step is further defined as injecting the plasmid bound to the carrier into the mammal intravenously.

4. A method as set forth in claim 3 wherein said injecting step is further defined as injecting the plasmid bound to the carrier intravenously into the blood stream of the mammal in proximity to the organ for transfection to increase the activity of the gene expression in the organ.

5. A method as set forth in claim 1 wherein said activating step is further defined as activating transient expression and cellular function by the inducer to transiently produce the desired gene products.

6. A method as set forth in claim 1 wherein the inducer is a foreign nucleic acid.

7. A method as set forth in claim 6 further including the step of promoting expression of particular expression vectors of the nucleic acid.

8. A method as set forth in claim 7 wherein said injecting step is further defined as injecting carriers containing plasmids containing a coding region for human growth hormone intravenously into a mouse, the region being driven by a mouse metallothionein promoter, said activating step being further defined as feeding the mouse a metallothionein promoter, the promoter activating the production of human growth hormone in the lung of the mouse.

9. A method as set forth in claim 7 wherein said injecting step is further defined as injecting a carrier containing a plasmid containing a prokaryotic gene, chloramphenicol acetyltransferace, into a mouse and activating chloramphenicol acetyltransferace activity in the lung cells of the mouse.

10. A method as set forth in claim 9 wherein said injecting step is further defined as injecting the carrier containing the plasmid intratracheally.
